# EUROPÄISCHE PATENTSCHRIFT

(11) **EP 3 261 685 B1**
(45) Veröffentlichungstag und Bekanntmachung des Hinweises auf die Patenterteilung: **29.09.2021**
(21) Anmeldenummer: 16700215.3
(22) Anmeldetag: 11.01.2016
(51) Int. Cl.: A61L 27/42, A61L 27/58, A61C 8/00, A61F 2/28, A61B 17/80

(54) **KOLLAGENHALTIGE WUNDAUFLAGE SOWIE VERFAHREN ZU DEREN HERSTELLUNG**
COLLAGEN-CONTAINING WOUND DRESSING AND METHOD FOR THE PRODUCTION THEREOF
PANSEMENT À BASE DE COLLAGÈNE ET PROCÉDÉ POUR LE PRODUIRE

(30) Priorität: 24.02.2015 DE 102015102598; 16.11.2015 DE 102015119776
(43) Veröffentlichungstag der Anmeldung: 03.01.2018
(73) Patentinhaber: Botiss Biomaterials GmbH, 15806 Zossen (DE)
(72) Erfinder: BIELENSTEIN, Oliver, 10629 Berlin (DE); TADIC, Drazen, 14129 Berlin (DE)
(74) Vertreter: Friderichs, Gunther
(86) Internationale Anmeldenummer: PCT/EP2016/050364
(87) Internationale Veröffentlichungsnummer: WO 2016/134866

(56) Entgegenhaltungen:
- WO-A2-2015/185597
- JP-A- 2010 082 146

## Beschreibung

### Gebiet der Erfindung

Die Erfindung betrifft ein kollagenhaltiges Implantat, welches als Wundauflage verwendet wird. Weiter betrifft die Erfindung ein Verfahren zur Herstellung des Implantats.

### Hintergrund der Erfindung

Kollagenhaltige Wundauflagen sind aus der Praxis in verschiedenen Ausführungsvarianten bekannt. So gibt es insbesondere vliesartige Wundauflagen aus einem Kollagen, welche aus einer Suspension eines nativen Kollagens hergestellt werden. Gegenüber aus einem chemisch aufgelösten Kollagen hergestellten Implantaten haben diese in der Regel den Vorteil, dass sie gut biegsam und reißfest sind.

Weiter gibt es Wundauflagen, welche aus Spenderhaut, insbesondere tierischen Ursprungs, hergestellt werden. Es handelt sich dabei insbesondere um Spalthaut porcinen Ursprungs, welche mittels nasschemischer Behandlung insbesondere mittels Ansäuerung und oxidativer Behandlung derart aufbereitet ist, dass im Wesentlichen ein keimfreies Kollagengerüst zurückbleibt. Derartige Wundauflagen werden insbesondere bei Brandverletzungen verwendet.

Nachteilig an derartig bekannten Implantaten ist, dass diese im gebogenen Zustand nicht formstabil sind, das heißt je nach Einsatzort kann das Implantat dazu neigen, sich unerwünscht von der Wunde abzurollen oder in eine Defektstelle, welche das Implantat überdeckt, einzusinken.

Dies betrifft insbesondere die Verwendung derartiger Kollagenimplantate im Kieferbereich, wo enge Biegeradien notwendig sind und die Kollegenimplantate oft verwendet werden, um das Einwachsen von Weichgewebe in eine Defektstelle eines Knochens, welches ggf. zuvor mit einem Knochenersatzmaterial aufgefüllt wurde, zu verhindern.

Das Dokument JP 2010 082146 A zeigt eine poröse Membran, welche mit einem Gerüst auf einer Magnesiumlegierung versehen ist.

### Aufgabe der Erfindung

Der Erfindung liegt demgegenüber die Aufgabe zugrunde, die genannten Nachteile des Standes der Technik zumindest zu reduzieren.

Es ist insbesondere eine Aufgabe der Erfindung, eine Wundauflage bereitzustellen, deren Formstabilität, insbesondere in gebogenem Zustand, verbessert ist.

### Zusammenfassung der Erfindung

Die Aufgabe der Erfindung wird bereits durch ein Verfahren zum Herstellen eines Implantats sowie durch ein Implantat nach einem der unabhängigen Ansprüche gelöst.

Bevorzugte Ausführungsformen und Weiterbildungen der Erfindung sind dem Gegenstand der jeweiligen Unteransprüche zu entnehmen.

Die Erfindung betrifft ein Verfahren zum Herstellen eines Implantats, welches als Wundauflage verwendet wird.

Gemäß der Erfindung wird ein Flächengebilde aus Magnesium oder einer Magnesiumlegierung, welches Aussparungen aufweist, mit einer kollagenhaltigen Zubereitung bedeckt und die kollagenhaltige Zubereitung wird getrocknet, wobei die Wundauflage als formstabile bioresorbierbare Membran ausgebildet ist. So entsteht ein Verbundmaterial aus Kollagen und Magnesium, bei welchem das Kollagen das Magnesium zumindest teilweise umschließt.

Unter einem Flächengebilde wird insbesondere eine Folie, ein Netz, ein Gewebe, insbesondere ein Vlies oder Gestrick verstanden. Dieses hat insbesondere eine Fläche von mehr als einem halben cm² und kann vom Benutzer gebogen werden. Bevorzugt wird eine Folie aus Magnesium oder einer Magnesiumlegierung verwendet, welche auch Aussparungen aufweisen kann, insbesondere welche als Netz ausgebildet ist.

Durch das Magnesium wird eine verbesserte Formstabilität des Verbundmaterials erreicht. Insbesondere neigt dieses weniger dazu, in überdeckte Defektstellen einzusinken. Gleichzeitig ist Magnesium bzw. eine Magnesiumlegierung ein bioresorbierbares Material, welches sich nach dem Einsetzen innerhalb gewisser Zeit abbaut, das heißt das Implantat braucht nicht nach dem Einsetzen entfernt zu werden.

Das Bedecken des Flächengebildes aus Magnesium bzw. einer Magnesiumlegierung kann auf beliebige Weise erfolgen, so insbesondere durch Abdecken des Flächengebildes mit der Zubereitung, durch Eintauchen, Übergießen oder durch Aufsprühen.

Das Flächengebilde aus Magnesium oder einer Magnesiumlegierung weist Aussparungen auf und ist insbesondere ein Flächengebilde, welches als Netz ausgebildet ist.

Bei letztgenannter Ausführungsvariante wäre sodann auch das Flächengebilde, welches sich aus der Kollagensuspension ausbildet, ausgespart und würde ebenfalls netzartig ausgebildet sein, da es das darunterliegende Gerüst aus Magnesium oder einer Magnesiumlegierung lediglich mit einem Flaum bedeckt. Die so aufgebrachte Suspension kann insbesondere als Haftvermittler zu einer weiteren Schicht, insbesondere zu einer Kollagenmembran, dienen.

Die kollagenhaltige Zubereitung ist flüssig und verfestigt sich durch Trocknung, insbesondere durch Lyophilisieren. Als kollagenhaltige Zubereitung wird insbesondere eine kollagenhaltige Suspension verwendet. Vorzugsweise wird ein natives Kollagen verwendet, also ein Kollagen, bei welchem die Kollagenfibrillen überwiegend erhalten und nicht chemisch aufgelöst wurden. Insbesondere wird ein natives Typ I-Kollagen verwendet. Eine derartige Kollagensuspension kann insbesondere aus einer Spenderhaut, vorzugsweise porcinen Ursprungs, hergestellt werden. Die Haut kann dabei mechanisch, beispielsweise durch Wolfen, zerkleinert werden und wird des Weiteren nasschemisch, vorzugsweise mittels Säurebehandlung und oxidativer Behandlung, aufbereitet, so dass insbesondere Fett überwiegend entfernt wird und ein im wesentliches keimfreies Ausgangsmaterial zurückbleibt.

Es wird insbesondere eine Kollagensuspension mit einem Feststoffanteil von 0,5 bis 5 % (Angaben immer in Gewichtsprozent) verwendet.

Für die Säurebehandlung kann insbesondere Salzsäure oder Phosphorsäure verwendet werden. Vorzugweise wird eine Chlorid-Ionen frei Säure, also insbesondere Phosphorsäure verwendet, da ansonsten die Korrosion des Magnesium beschleunigt wird.

Das Flächengebilde aus Magnesium bzw. einer Magnesiumlegierung kann, wie es bei einer Ausführungsform der Erfindung vorgesehen ist, eine Beschichtung aufweisen, insbesondere eine Passivierung. Es ist insbesondere vorgesehen, ein Flächengebilde mit einer Passivierungsschicht aus Magnesiumfluorid zu verwenden. Diese kann erzeugt werden, indem das Flächengebilde in Flusssäure eingetaucht wird.

Vorzugsweise wird die kollagenhaltige Zubereitung zumindest vor dem Bedecken des Flächengebildes aus Magnesium oder einer Magnesiumlegierung neutralisiert, insbesondere auf einen pH-Wert größer 5,5, vorzugsweise größer 6,5. So wird das Risiko des Auflösens des Magnesiums oder der Passivierungsschicht verhindert.

Das Neutralisieren erfolgt mit einem basischen Puffer, insbesondere mit einem Phosphatpuffer, wie Trinatriumphosphat.

Das Flächengebilde aus Magnesium oder einer Magnesiumlegierung kann insbesondere als Netz ausgebildet werden. Vorzugsweise ist das Flächengebilde als Folie mit Aussparungen ausgebildet. Gegenüber einem beispielsweise aus Drähten gestrickten Netz führt dies zu einer besseren Formstabilität.

Insbesondere wird als Grundmaterial eine 20 bis 300 µm dicke Magnesiumfolie verwendet, in welche Aussparungen eingebracht werden.

Die Aussparungen dienen der Ausgestaltung eines verbesserten Verbunds.

Bei einer Weiterbildung der Erfindung wird das Flächengebilde aus Magnesium oder einer Magnesiumlegierung auf einen Träger aufgebracht.

Als Träger dient insbesondere eine Kollagenmembran, wie beispielsweise eine nasschemisch aufbereitete und lyophilisierte Haut tierischen oder humanen Ursprungs. Es kann insbesondere eine nasschemisch aufbereitete Spalthaut porcinen Ursprungs verwendet werden.

Nach dem Aufbringen des Flächengebildes aus Magnesium oder einer Magnesiumlegierung auf den Träger wird vorzugsweise das Flächengebilde mit der kollagenhaltigen Zubereitung bedeckt und es bildet sich ein Verbundmaterial aus drei Komponenten, nämlich dem Träger, dem auf dem Träger angeordneten Kollagenvlies, welches durch Trocknung der Zubereitung entstanden ist sowie dem Flächengebilde aus Magnesium oder einer Magnesiumlegierung, welches in dem Kollagenvlies eingebettet und gleichzeitig mit dem Träger verbunden ist.

Insbesondere durch Verwendung einer kollagenhaltigen Suspension kann so ein Verbund hergestellt werden, bei dem es nicht nötig ist, das Flächengebilde aus Magnesium oder einer Magnesiumlegierung mit dem Träger durch zusätzliche Mittel zu verbinden.

Es ist aber dennoch denkbar, zur Verbesserung des Zusammenhaftens des Verbunds den Träger beispielsweise durch Vernähen mit dem Flächengebilde aus Magnesium oder einer Magnesiumlegierung zu verbinden.

Weiter kann das Flächengebilde aus Magnesium oder einer Magnesiumlegierung eine Strukturierung aufweisen, um den Verbund zu verbessern, beispielsweise in Form einer Aufrauung oder in Form von kleinen Widerhaken.

Denkbar ist aber auch, das Flächengebilde aus Magnesium oder einer Magnesiumlegierung in eine Art Tasche einzubringen. So kann insbesondere eine Spalthaut hergestellt werden, welche sodann aus einer oberen und einer unteren Kollagenmembran besteht. In die so gebildete Tasche wird das Flächengebilde aus Magnesium oder einer Magnesiumlegierung eingelegt.

Weiter ist denkbar, das Flächengebilde aus Magnesium oder einer Magnesiumlegierung zwischen zwei Kollagenmembranen anzuordnen. Insbesondere könnte aus zwei Kollagenmembranen, etwa durch Vernähen, eine Tasche gebildet werden. Diese Ausführungsform der Erfindung schließt nicht aus, dass das Flächengebilde aus Magnesium oder einer Magnesiumlegierung vor dem Einlegen zwischen die Kollagenmembranen mit einer Kollagensuspension bedeckt wird, welche sodann als Haftvermittler zu den angrenzenden Membranen dient. Insbesondere wird das mit der noch nassen Kollagensuspension beschichtete Flächengebilde aus Magnesium oder einer Magnesiumlegierung zwischen die beiden Kollagenmembranen eingebracht, ggf. werden die Kollagenmembranen sodann noch zusammengedrückt und der entstandene Verbund wird lyophilisiert.

Das durch die Erfindung bereitgestellte Verbundmaterial sollte eine Dicke von weniger als 4, vorzugsweise weniger als 3 mm aufweisen.

Die Erfindung betrifft des Weiteren ein Implantat, welches wie vorstehend beschrieben herstellbar ist.

Das Implantat umfasst ein Flächengebilde aus Magnesium bzw. einer Magnesiumlegierung. Das Flächengebilde ist mit einem kollagenhaltigen Flächengebilde verbunden, insbesondere ist das Flächengebilde aus Magnesium oder einer Magnesiumlegierung in dem kollagenhaltigen Flächengebilde eingebettet.

Bei dem kollagenhaltigen Flächengebilde handelt es sich insbesondere auch um ein Kollagenvlies. Dieses kann wie vorstehend beschrieben durch Lyophilisation einer Suspension eines nativen Kollagens hergestellt werden.

Das kollagenhaltige Flächengebilde weist Aussparungen auf. Insbesondere wenn ein netzartiges Flächengebilde aus Magnesium oder einer Magnesiumlegierung verwendet wird, kann dies lediglich mit einem Flaum aus einem Kollagenvlies bedeckt sein.

Bei einer Weiterbildung der Erfindung umfasst das Implantat einen Träger, insbesondere einen Träger aus einer Kollagenmembran, auf welchem das kollagenhaltige Flächengebilde angeordnet ist.

Das Flächengebilde aus Magnesium oder einer Magnesiumlegierung weist gemäß der Erfindung zumindest abschnittsweise Aussparungen auf und ist insbesondere so als Netz ausgebildet.

Das Flächengebilde aus Magnesium oder einer Magnesiumlegierung hat vorzugsweise eine Dicke von 20 µm bis 1 mm, besonders bevorzugt von 50 µm bis 300 µm. Das Implantat ist insbesondere gekrümmt ausgebildet und derart formstabil, dass es nicht in Defektstellen einsinkt.

### Kurzbeschreibung der Zeichnungen

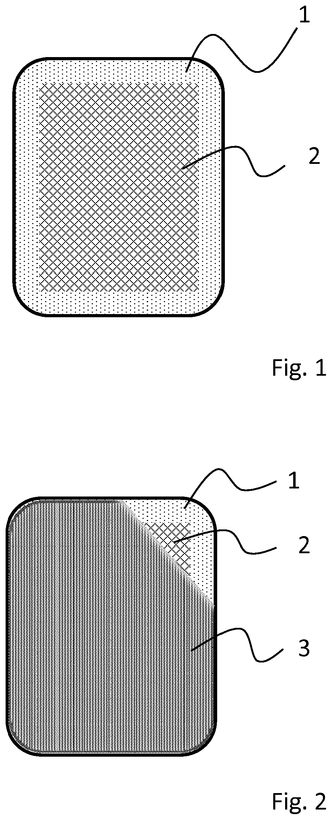

Der Gegenstand der Erfindung soll im Folgenden bezugnehmend auf die Zeichnungen Fig. 1 bis Fig. 4 näher erläutert werden.

Bezugnehmend auf Fig. 1 bis Fig. 3 soll eine beispielhafte Herstellung eines erfindungsgemäßen Implantats erläutert werden.

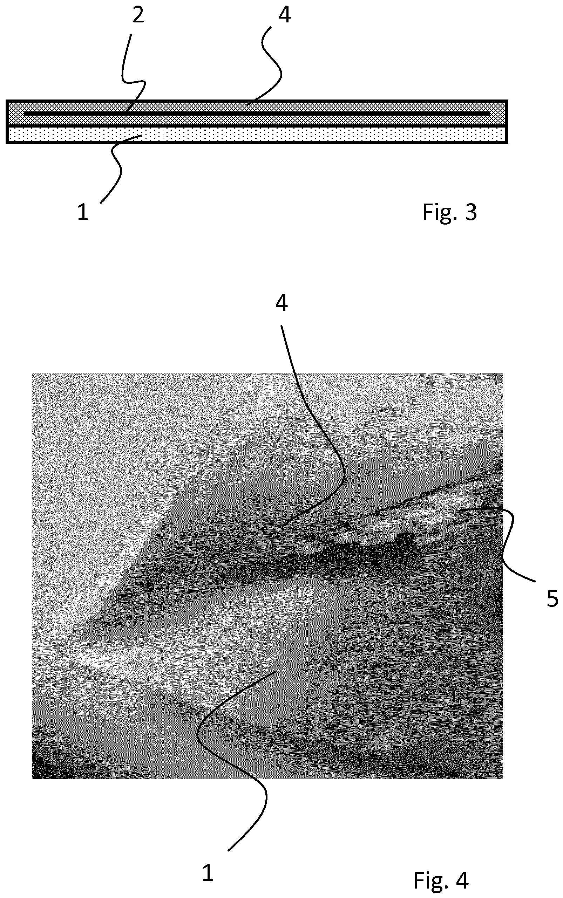

- Fig. 4: zeigt ein Lichtbild eines Implantats.

### Detaillierte Beschreibung der Zeichnungen

Fig. 1 zeigt in einer schematischen Darstellung einen Träger 1. Dieser besteht in diesem Ausführungsbeispiel aus einer nasschemisch aufbereiteten Spalthalt porcinen Ursprungs. Es handelt sich mithin um eine Kollagenmembran.

Auf den Träger 1 aufgelegt wurde ein Flächengebilde aus Magnesium oder einer Magnesiumlegierung 2.

Das Flächengebilde ist in diesem Ausführungsbeispiel als Netz ausgebildet, welches aus einer Folie aus Magnesium bzw. einer Magnesiumlegierung besteht. Die Aussparungen des Netzes verlaufen in diesem Ausführungsbeispiel rautenförmig.

Das Flächengebilde aus Magnesium oder einer Magnesiumlegierung 2 reicht nicht bis ganz an den Rand des Trägers 1.

Wie nunmehr in Fig. 2 dargestellt, wird Träger 1 und Flächengebilde aus Magnesium oder einer Magnesiumlegierung mit einer kollagenhaltigen Zubereitung 3 bedeckt.

In diesem Ausführungsbeispiel handelt es sich um eine 0,5 bis 5 %ige Kollagensuspension porcinen Ursprungs. Die Kollagensuspension wird vor dem Aufbringen mittels eines Phosphatpuffers neutralisiert, insbesondere ein pH-Wert von 7 +/- 0,5 eingestellt.

Die kollagenhaltige Zubereitung 3 wird sodann mit einem geeigneten Handhabungswerkzeug verstrichen.

Sodann wird das so hergestellte Verbundmaterial für einen Zeitraum von 15 bis 120 Minuten ruhen gelassen, bis der Träger 1 gut durchfeuchtet ist.

Im Anschluss erfolgt eine Lyophilisation über einen Zeitraum von 6 Stunden bis 5 Tagen.

Fig. 3 zeigt nunmehr eine schematische Schnittansicht des hergestellten Verbundmaterials.

Dieses besteht aus dem Träger 1, auf welchem nunmehr das Flächengebilde aus Magnesium oder einer Magnesiumlegierung 2 in einem Kollagenvlies 4, welches sich aus der kollagenhaltigen Zubereitung gebildet hat, eingebettet ist.

Das Kollagenvlies 4 sorgt zugleich für einen festen Verbund mit dem Träger 1.

Fig. 4 zeigt ein Lichtbild eines erfindungsgemäßen Verbundmaterials, bei welchem die verschiedenen Schichten auseinandergerissen sind.

Zu erkennen ist der als Kollagenmembran ausgebildete Träger sowie das davon abgerissene Kollagenvlies 4, in welchem ein Netz 5 aus Magnesium eingebettet ist.

Durch die Erfindung konnte auf einfache Weise eine formstabile bioresorbierbare Membran bereitgestellt werden, welche sich insbesondere auch zum Abdecken von Defektstellen im Knochen/Kieferbereich eignet, ohne dass das Verbundmaterial dazu neigt, einzusinken.

## Patentansprüche

1. Verfahren zum Herstellen einer Wundauflage, wobei ein Flächengebilde aus Magnesium oder einer Magnesiumlegierung, welches Aussparungen aufweist, mit einer kollagenhaltigen Zubereitung bedeckt wird und die kollagenhaltige Zubereitung getrocknet wird, und die Wundauflage als formstabile bioresorbierbare Membran ausgebildet ist.

2. Verfahren zum Herstellen einer Wundauflage nach dem vorstehenden Anspruch, **dadurch gekennzeichnet, dass** als kollagenhaltige Zubereitung eine kollagenhaltige Suspension verwendet wird.

3. Verfahren zum Herstellen einer Wundauflage nach einem der vorstehenden Ansprüche, **dadurch gekennzeichnet, dass** eine Suspension mit einem nativen Kollagen verwendet wird.

4. Verfahren zum Herstellen einer Wundauflage nach einem der vorstehenden Ansprüche, **dadurch gekennzeichnet, dass** das Flächenbilde aus Magnesium oder einer Magnesiumlegierung als Netz ausgebildet ist.

5. Verfahren zum Herstellen einer Wundauflage nach einem der vorstehenden Ansprüche, **dadurch gekennzeichnet, dass** das Flächengebilde aus Magnesium oder einer Magnesiumlegierung auf einen Träger aufgebracht wird.

6. Verfahren zum Herstellen einer Wundauflage nach dem vorstehenden Anspruch, **dadurch gekennzeichnet, dass** der Träger eine Kollagenmembran, insbesondere eine lyophilisierte Haut tierischen oder humanen Ursprungs, verwendet wird.

7. Verfahren zum Herstellen einer Wundauflage nach einem der vorstehenden Ansprüche, **dadurch gekennzeichnet, dass** die kollagenhaltige Zubereitung zumindest vor dem Bedecken des Flächengebildes aus Magnesium oder einer Magnesiumlegierung auf einen pH-Wert größer 5,5, vorzugsweise größer 6,5 eingestellt wird.

8. Verfahren zum Herstellen einer Wundauflage nach einem der vorstehenden Ansprüche, **dadurch gekennzeichnet, dass** das Trocknen der kollagenhaltigen Zubereitung durch Lyophilisation erfolgt.

9. Wundauflage, herstellbar mit einem Verfahren nach einem der vorstehenden Ansprüche, umfassend ein Flächengebilde aus Magnesium oder einer Magnesiumlegierung, welches Aussparungen aufweist, welches mit einem kollagenhaltigen Flächengebilde verbunden ist, insbesondere in dem kollagenhaltigen Flächengebilde eingebettet ist, und wobei die Wundauflage als formstabile bioresorbierbare Membran ausgebildet ist.

10. Wundauflage nach dem vorstehenden Anspruch, **dadurch gekennzeichnet, dass** das Implantat einen Träger, insbesondere einen Träger aus einer Kollagenmembran, umfasst, auf welchem das kollagenhaltige Flächengebilde angeordnet ist.

11. Wundauflage nach einem der Ansprüche 9 bis 10, **dadurch gekennzeichnet, dass** das Flächengebilde aus Magnesium oder einer Magnesiumlegierung als Netz ausgebildet ist.

12. Wundauflage nach einem der Ansprüche 9 bis 11, **dadurch gekennzeichnet, dass** das Flächengebilde aus Magnesium oder einer Magnesiumlegierung eine Dicke von von 20 µm bis 1 mm, vorzugsweise von 50 µm bis 300 µm aufweist.

13. Wundauflage nach einem der Ansprüche 9 bis 12, **dadurch gekennzeichnet, dass** die Wundauflage gekrümmt ausgebildet ist.

14. Wundauflage nach einem der Ansprüche 9 bis 13, **dadurch gekennzeichnet, dass** das kollagenhaltige Flächengebilde als Vlies und/oder als Kollagenmembran ausgebildet ist.

## Claims

1. A method for producing a wound dressing, wherein a sheet-like structure made of magnesium or of a magnesium alloy, which comprises openings, is covered with a collagen-containing preparation, and the collagen-containing preparation is dried, and wherein the wound dressing is embodied as a geometrically stable bioresorbable membrane.

2. The method for producing an implant as claimed in the preceding claim, wherein a collagen-containing suspension is used as the collagen-containing preparation.

3. The method for producing an implant as claimed in the preceding claim, wherein a suspension including a native collagen is used.

4. The method for producing an implant as claimed in any of the preceding claims, wherein the sheet-like structure made of magnesium or of a magnesium alloy is in the form of a mesh.

5. The method for producing an implant as claimed in any of the preceding claims, wherein the sheet-like structure made of magnesium or of a magnesium alloy is applied onto a support.

6. The method for producing an implant as claimed in the preceding claim, wherein a collagen membrane is used as the support, in particular a lyophilized skin of animal or human origin.

7. The method for producing an implant as claimed in any of the preceding claims, wherein the collagen-containing preparation is adjusted to a pH value of greater than 5.5, preferably greater than 6.5, at least prior to the covering of the sheet-like structure made of magnesium or of a magnesium alloy.

8. The method for producing an implant as claimed in any of the preceding claims, wherein the drying of the collagen-containing preparation is accomplished by lyophilization.

9. Wound dressing, particular preparable by a method according to any of the preceding claims, comprising a sheet-like structure made of magnesium or of a magnesium alloy, which comprises openings and which is joined to a collagen-containing sheet-like structure, in particular embedded in the collagen-containing sheet-like structure, and wherein the wound dressing is embodied as a geometrically stable bioresorbable membrane.

10. The wound dressing as claimed in the preceding claim, wherein the implant comprises a support, in particular a support made of a collagen membrane, on which the collagen-containing sheet-like structure is arranged.

11. The wound dressing as claimed in any of the claims 9 to 10, wherein the sheet-like structure made of magnesium or of a magnesium alloy has openings, at least in sections thereof, and in particular is in the form of a mesh.

12. The wound dressing as claimed in any of the claims 9 to 11, wherein the sheet-like structure made of magnesium or of a magnesium alloy has a thickness from 20 µm to 1 mm, preferably from 50 µm to 300 µm.

13. The wound dressing as claimed in any of the claims 9 to 12, wherein the wound dressing is curved.

14. The implant as claimed in any of the claims 9 to 13, wherein the collagen-containing sheet-like structure is in the form of a non-woven fabric and/or a collagen membrane.

## Revendications

1. Procédé de fabrication d'un pansement, dans lequel une structure plane en magnésium ou en alliage de magnésium, présentant des creux, est recouverte d'une préparation contenant du collagène, la préparation contenant du collagène est séchée et le pansement est conçu comme membrane biorésorbable de forme stable.

2. Procédé de fabrication d'un pansement selon la revendication précédente, **caractérisé en ce qu'**une suspension contenant du collagène est utilisée comme préparation contenant du collagène.

3. Procédé de fabrication d'un pansement selon l'une des revendications précédentes, **caractérisé en ce qu'**une suspension contenant un collagène natif est utilisée.

4. Procédé de fabrication d'un pansement, selon l'une des revendications précédentes, **caractérisé en ce que** la structure plane en magnésium ou en alliage de magnésium est conçue comme filet.

5. Procédé de fabrication d'un pansement, selon l'une des revendications précédentes, **caractérisé en ce que** la structure plane en magnésium ou en alliage de magnésium est déposée sur un support.

6. Procédé de fabrication d'un pansement, selon la revendication précédente, **caractérisé en ce que** le support utilise une membrane en collagène, en particulier une membrane lyophilisée d'origine animale ou humaine.

7. Procédé de fabrication d'un pansement, selon l'une des revendications précédentes, **caractérisé en ce que** la préparation contenant du collagène est portée à un pH supérieur à 5,5, préférablement supérieur à 6,5, au moins avant la couverture de la structure plane en magnésium ou en alliage de magnésium.

8. Procédé de fabrication d'un pansement, selon l'une des revendications précédentes, **caractérisé en ce que** le séchage de la préparation contenant du collagène s'effectue par lyophilisation.

9. Pansement, pouvant être fabriqué au moyen d'un procédé selon l'une des revendications précédentes, comprenant une structure plane en magnésium ou en alliage de magnésium, présentant des creux, laquelle est reliée à une structure plane contenant du collagène, en particulier intégrée dans la structure plane contenant du collagène, et dans lequel le pansement est conçu comme membrane biorésorbable de forme stable.

10. Pansement selon la revendication précédente, **caractérisé en ce que** l'implant comprend un support, en particulier un support constitué d'une membrane en collagène, sur lequel est disposée la structure plane contenant du collagène.

11. Pansement selon l'une des revendications 9 à 10, **caractérisé en ce que** la structure plane en magnésium ou en alliage de magnésium est conçue comme filet.

12. Pansement selon l'une des revendications 9 à 11, **caractérisé en ce que** la structure plane en magnésium ou en alliage de magnésium présente une épaisseur de 20 pm à 1 mm, préférablement de 50 µm à 300 µm.

13. Pansement selon l'une des revendications 9 à 12, **caractérisé en ce que** le pansement est de forme incurvée.

14. Pansement selon l'une des revendications 9 à 13, **caractérisé en ce que** la structure plane contenant du collagène est conçue comme matière non-tissée et/ou comme membrane en collagène.
